# EUROPÄISCHE PATENTSCHRIFT

(11) **EP 3 709 859 B1**
(45) Veröffentlichungstag und Bekanntmachung des Hinweises auf die Patenterteilung: **22.09.2021**
(21) Anmeldenummer: 18803944.0
(22) Anmeldetag: 14.11.2018
(51) Int. Cl.: A61B 1/00, A61B 17/00, A61B 17/29, A61B 17/3205, A61B 1/307, A61B 17/221

(54) **MEDIZINISCHES GERÄT ZUM BEWEGEN EINES MEDIZINISCHEN INSTRUMENTS**
MEDICAL DEVICE FOR MOVING A MEDICAL INSTRUMENT
APPAREIL MÉDICAL POUR DÉPLACER UN INSTRUMENT MÉDICAL

(30) Priorität: 14.11.2017 DE 102017010535
(43) Veröffentlichungstag der Anmeldung: 23.09.2020
(73) Patentinhaber: UROMED KURT DREWS KG, 22113 Oststeinbek (DE)
(72) Erfinder: SCHWARZ, Werner, 83324 Ruhpolding (DE)
(74) Vertreter: Eisenführ Speiser
(86) Internationale Anmeldenummer: PCT/EP2018/081207
(87) Internationale Veröffentlichungsnummer: WO 2019/096835

(56) Entgegenhaltungen:
- WO-A1-2017/153178
- DE-T2- 69 433 774
- US-A1- 2003 009 176
- US-A1- 2010 191 224

## Beschreibung

Die Erfindung bezieht sich auf ein medizinisches Gerät, vorzugsweise ein Gerät zur Verwendung in einem medizinischen Endoskop, zum Bewegen eines medizinischen Instruments, vorzugsweise eines Steinfanginstruments, nach dem Oberbegriff von Anspruch 1.

Ein medizinisches Gerät der vorstehend genannten Art ist bereits bekannt (siehe im Internet: https://www.youtube.com/watch?v=2BhfMzx5pNE). Dieses bekannte Gerät erfordert zu seiner Bedienung eine Zwei-Hand-Bedienung oder ein Umgreifen: durch Betätigen einer ersten Hand wird ein Steinfangkörbchen aus dem Gerät herausgefahren oder in dieses eingefahren, und durch Betätigen einer zweiten Hand wird das Steinfangkörbchen geöffnet oder geschlossen. Zuweilen wird eine solche Gerätebedienung jedoch als zu aufwendig empfunden. Einen solchen Stand der Technik zeigt die US 2003/0009176 A1. Mittels eines Schiebeschalters 31 ist eine Verschiebung eines Steinfangkörbchen in Gerätelängsrichtung möglich. Mit einem davon beabstandet angeordneten Drehrad 24 ist ein Drehen des Steinfangkörbchens um die Gerätelängsrichtung herum möglich.

Die US 2010/0191224 A1 betrifft ein Griffteil für ein Steinfanginstrument, das unter anderem eine Betätigungseinrichtung zum Öffnen und Schließen eines Steinfangkörbchens offenbart. Zusätzlich zu dem Schiebeschalter kann zur Betätigung auch ein seitlich daran angeordnetes Stellrad verwendet werden. Beide Betätigungselemente - Schiebeschalter und Stellrad - bewirken ausschließlich eine Bewegung in Gerätelängsrichtung. Weitere Betätigungseinrichtungen offenbaren die WO 2017/153178 A1 und die DE 694 33 774 T2.

Der Erfindung liegt daher die Aufgabe zu Grunde, ein medizinisches Gerät der eingangs genannten Art so aufzubauen, dass das medizinische Instrument auf relativ einfache Weise mittels einer Betätigungseinrichtung durch eine Einhandbedienung sowohl in Gerätelängsrichtung als auch in Drehrichtung quer um die betreffende Längsrichtung bewegbar ist.

### Beschreibung

Gelöst wird die vorstehend aufgezeigte Aufgabe bei einem medizinischen Gerät der eingangs genannten Art erfindungsgemäß durch die Merkmale des Anspruchs 1.

Dabei ist das "medizinische Gerät" vorzugsweise ein Gerät zur Verwendung in einem medizinischen Endoskop, das beispielsweise zur Entfernung von Nierensteinen, Steinen im Ureter oder anderen Konkrementen eingesetzt werden kann. Zur Hindurchführung von Durchgangsinstrumenten weist das medizinische Gerät einen langgestreckten Außentubus auf.

Das "medizinische Instrument" ist ein Durchgangsinstrument, das in dem Außentubus des medizinischen Geräts aufgenommen werden kann bzw. diesen durchläuft, d.h. das im Inneren des Außentubus des medizinischen Geräts angeordnet ist, vorzugsweise ein Steinfanginstrument. Das medizinische Instrument ist in dem medizinischen Gerät, insbesondere in dem Außentubs, axial verschiebbar (längsverschiebbar) und um seine Längsachse drehbar gelagert. Die axiale Verschiebung und die Drehung um die Längsachse des Instruments werden mittels der hierin beschriebenen Betätigungseinrichtung bewirkt.

Die Erfindung bringt den Vorteil mit sich, dass sich das medizinische Instrument auf relativ einfache Weise mittels einer Betätigungseinrichtung durch eine Einhandbedienung sowohl in Gerätelängsrichtung als auch in Drehrichtung quer um die betreffende Längsrichtung bewegen lässt.

Die erfindungsgemäße Verwendung von zwei magnetisch gekoppelten Elementen hat den besonderen zusätzlichen Vorteil, dass die zusammenwirkenden magnetischen Elemente auch als Sicherungseinrichtung wirken, die eine Zerstörung des distalen Arbeitselements durch eine zu starke Krafteinwirkung durch den Benutzer verhindern. So kann es in handelsüblichen Instrumenten zu einer Beschädigung des Arbeitselements (z. B. des Steinfangkörbchens) kommen, wenn der Benutzer nach dem Einfangen des Konkrements das Arbeitselement unter zu starker Krafteinwirkung in proximale Richtung in den Außentubus zurückzieht. Werden das erfindungsgemäß zusammenwirkende erste und zweite magnetische Element verwendet, so wird in einem solchen Fall überschießender Krafteinwirkung das magnetische Zusammenwirken der Elemente unterbrochen und das Betätigungselement unter Auflösung des magnetischen Zusammenwirkens weiter in proximale Richtung verschoben.

Bei Vorhandensein einer mechanischen Koppelung zwischen dem zumindest einen zweiten magnetischen Element und dem zumindest einen ersten magnetischen Element in der genannten Gerätelängsrichtung lässt sich das zumindest eine erste magnetische Element durch magnetische Koppelung lediglich in der genannten Drehrichtung bewegen. Ohne eine solche mechanische Koppelung lässt sich das zumindest eine erste magnetische Element indessen sowohl in der genannten Gerätelängsrichtung als auch in der betreffenden Drehrichtung durch magnetische Koppelung bewegen.

Vorzugsweise ist gemäß einer zweckmäßigen Weiterbildung der Erfindung der Betätigungseinrichtung eine Skaleneinrichtung bzw. ein Skalenelement zugeordnet, die/das das Ausmaß zumindest einer Bewegung des medizinischen Instruments in Gerätelängsrichtung und/oder in der Drehrichtung quer dazu anzuzeigen bzw. zu ermitteln gestattet. Von Vorteil kann es sein, das Ausmaß der Bewegung des medizinischen Instruments sowohl in der Gerätelängsrichtung als auch in der Drehrichtung quer dazu, also mittels zweier Skalen anzuzeigen und damit diese Ausmaße in zwei Koordinaten zu ermitteln. Dies ist insbesondere in Fällen von Nutzen, in denen mit dem betreffenden medizinischen Instrument aus Hohlräumen eines Individuums Körperteile oder Konkremente, zum Beispiel Gallen- oder Nierensteine herausgeholt werden sollen. Dabei ist es von Vorteil, vor einem solchen Herausholen zumindest über eine ungefähre Information darüber zu verfügen, von welcher Größe der jeweils zu entfernende Körperteil bzw. das jeweilige Konkrement ist. Dies heißt, dass die genannte Skaleneinrichtung bzw. das genannte Skalenelement gewissermaßen zum Ausmessen der jeweils zu entfernenden Körperteile bzw. des jeweiligen Konkrements herangezogen werden kann.

Gemäß einer anderen zweckmäßigen Weiterbildung der Erfindung umfasst das vorliegende medizinische Gerät folgende Merkmale:
- das medizinische Gerät weist ein seine Handhabung ermöglichendes oder zumindest erleichterndes proximales Ende bzw. Endbereich und ein das medizinische Instrument enthaltendes distales Ende bzw. Endbereich auf,
- das medizinische Instrument weist ein von dem distalen Ende des Geräts abstehendes oder aus diesem herausführbares Arbeitsende auf und ist mit seinem an dieses Arbeitsende zum proximalen Ende des Gerätes hin sich anschließenden Bereich von einem Instrumenten-Aufnahmekörper sowohl in der Gerätelängsrichtung als auch in Drehrichtung quer dazu verschiebbar,
- in dem Instrumenten-Aufnahmekörper ist das wenigstens eine erste magnetische Element mit dem medizinischen Instrument gekoppelt,
- und das wenigstens eine erste magnetische Element ist von dem wenigstens einen zweiten magnetischen Element umgeben, welches in Bezug auf das wenigstens eine erste magnetische Element in Gerätelängsrichtung und in Drehrichtung quer dazu bewegbar ist und welches mit dem wenigstens einen ersten magnetischen Element magnetisch zusammenwirkt.

Hierdurch ergibt sich der Vorteil eines insgesamt besonders einfach aufzubauenden medizinischen Geräts gemäß der Erfindung.

Erfindungsgemäß ist das wenigstens eine zweite magnetische Element von einem Drehschieber aufgenommen, der zusammen mit dem wenigstens einen zweiten magnetischen Element um das wenigstens eine erste magnetische Element sowohl in der Gerätelängsrichtung als auch in Drehrichtung quer dazu verschiebbar ist. Hierdurch lässt sich das wenigstens eine zweite magnetische Element in vorteilhafter Weise besonders einfach relativ zu dem wenigstens einen ersten magnetischen Element sowohl in Gerätelängsrichtung als auch in Drehrichtung quer dazu bewegen.

Vorzugsweise ist der Drehschieber zwischen dem Aufnahmekörper und einem in der Gerätelängsrichtung proximal vorgesehenen, als Handauflagekörper nutzbaren Verlängerungsteil verschieb- und drehbar, der von dem Aufnahmekörper aufnehmbar ist. Diese Maßnahme erleichtert die Einhandbedienung des medizinischen Geräts gemäß der Erfindung.

Zweckmäßigerweise sind das wenigstens eine erste magnetische Element und das wenigstens eine zweite magnetische Element so angeordnet, dass sie mit magnetischen Gegenpolen zusammen wirken. Hierdurch ergibt sich der Vorteil einer besonders einfachen Realisierung der wenigstens einen ersten und zweiten magnetischen Elemente. Zweckmäßigerweise sind die magnetischen Elemente durch Stabmagneten gebildet, deren Gegenpole (Nord- und Südpole) jeweils in der Gerätelängsrichtung ausgerichtet sind. Alternativ dazu können die jeweiligen magnetischen Elemente durch Rundmagneten gebildet sein, deren Gegenpole (Nord- und Südpole) jeweils quer zu der Gerätelängsrichtung ausgerichtet sind. In beiden Fällen stehen so einfach aufzubauende magnetische Elemente zur Verfügung.

Vorzugsweise sind die jeweiligen magnetischen Elemente durch Permanentmagneten gebildet. Dies bringt den Vorteil besonders einfach zu realisierender magnetischer Elemente mit sich. Es sei an dieser Stelle jedoch angemerkt, dass es prinzipiell auch möglich ist, das eine oder andere oder sämtliche der magnetischen Elemente jeweils durch ein elektromagnetisches Element zu realisieren.

Als medizinisches Instrument dient vorzugsweise ein endoskopisch einsetzbares medizinisches Instrument, ein medizinisches Steinfangkörbchen oder eine medizinische Schlinge, wie eine Polypektomieschlinge. Damit ist die vorliegende Erfindung in einem weiten Gebiet der Medizin einsetzbar, wie zum Beispiel in Gallen- und Nierenbereichen und in der Gastroenterologie.

Das medizinische Instrument weist bevorzugt einen Schaftteil auf, der flexibel bzw. biegeelastisch ausgebildet ist. Die Flexibilität des Schaftteils erlaubt es, das Instrument durch den Außentubus eines medizinischen Geräts einzuführen und durch diesen Tubus bis zur Eingriffsstelle zu schieben. Das in Kombination mit dem Instrument verwendete Gerät weist bevorzugt ebenfalls ein flexibles Schaftteil auf, der durch den Außentubus gebildet werden kann oder einen solchen umfasst.

Das/der die Handhabung ermöglichende oder zumindest erleichternde proximale Ende bzw. Endbereich des medizinischen Geräts kann ein Handgriff sein oder einen Handgriff umfassen. Der Handgriff kann beispielsweise ein Abschlussgriffteil umfassen. Der Handgriff ist vorzugsweise zur einhändigen Bedienung ausgebildet. Dies bedeutet, dass alle im Handgriff implementierten Funktionen, einschließlich des Haltens des Geräts sowie der Betätigung des hierin beschriebenen Betätigungselements, mit einer Hand bedienbar sind.

In bevorzugten Ausführungsformen ist der Betätigungseinrichtung ein Skalenelement zugeordnet, das die Länge bzw. Größe eines im Steinfangkörbchen gefangenen Konkrements anzeigen kann. Hierzu wird eine auf dem Skalenelement angeordnete Skala durch Magnetkraft zwangsgeführt zwischen dem Drehschieber und dem Abdeckrohr in distale Richtung hervorgeschoben, wenn der Drehschieber maximal in proximale Richtung verschobenen ist. Dieser Effekt wird durch die Verwendung eines Skalenelements mit einem L-förmigen Querschnitt erreicht, das federvorgespannt in der Betätigungseinrichtung maximal in proximale Richtung verschoben ist, wobei die Skala in dieser Stellung für den Benutzer nicht sichtbar ist, da sie zwischen Drehschieber und Abdeckrohr angeordnet ist. Das zweite magnetische Element ist proximal des Skalenelements angeordnet. Ist ein Konkrement in dem Steinfangkörbchen aufgenommen und wird der Drehschieber vom Benutzer maximal in proximale Richtung verschoben, so hält die Magnetkraft des ersten magnetischen Elements das zweite magnetische Element in einer Position, die distal von seiner maximalen proximalen Position ist. Entsprechend wird das Federelement, das zur Ausbildung einer Vorspannung verwendet ist, komprimiert und die Skala zwischen dem Drehschieber und dem Abdeckrohr in distale Richtung hervorgeschoben, sodass die Skala für den Benutzer sichtbar wird. Somit ist erfindungsgemäß mittels einer Längsverschiebung des wenigstens einen zweiten magnetischen Elements in distale Richtung ein Skalenelement in distale Richtung verschiebbar.

Dazu weist das Skalenelement ein erstes Teil auf, das eine Skala umfasst und das parallel zur Längsrichtung des medizinischen Instruments bzw. parallel zu dessen Schaftbereich angeordnet ist. Das erste Teil kann beispielsweise auf dem Abdeckrohr des medizinischen Geräts aufliegen und zu diesem mindestens abschnittsweise formkomplementär sein. So kann das erste Teil beispielsweise einen teilkreisförmigen Querschnitt aufweisen, der der zylindrischen Form des Abdeckrohrs angepasst ist. Das erste Teil des Skalenelements ist mindestens abschnittsweise zwischen dem Abdeckrohr und der Betätigungseinrichtung bzw. dem Drehschieber der Betätigungseinrichtung angeordnet. Am proximalen Ende des ersten Teils ist ein zweites Teil des Skalenelements angeordnet.

Das zweite Teil des Skalenelements ist im Wesentlichen orthogonal zur Längsrichtung des ersten Teils angeordnet und damit auch im Wesentlichen orthogonal zur Längsrichtung des medizinischen Instruments. Das Skalenelement kann beispielsweise aus einem im Wesentlichen L-förmigen Element, zum Beispiel einem Blech-, Kunststoff- oder Keramikelement gebildet werden. Das kurze Ende der L-Form würde in diesem Falle das zweite Teil des Skalenelements bilden und das lange Ende das erste Teil. "L-Form" bezeichnet in diesem Zusammenhang eine Form mit einem L-förmigen Querschnitt. Das Skalenelement ist vorzugsweise aus einem Stück gebildet. Jedenfalls aber sind das erste und das zweite Teil des Skalenelements relativ zueinander nicht verschiebbar.

Das zweite Teil des Skalenelements ist vollständig zwischen dem Drehschieber und dem Abdeckrohr angeordnet. Distal des zweiten Teils - und vorzugsweise radial neben einem Abschnitt des ersten Teils - ist ein Federelement angeordnet. Das Federelement übt auf das zweite Teil eine Vorspannung aus distaler Richtung aus. Proximal des zweiten Teils ist das zweite magnetische Element angeordnet. Mit anderen Worten ist das zweite Teil des Skalenelements somit zwischen dem Federelement und dem zweiten magnetischen Element angeordnet. Mittels einer Längsverschiebung des wenigstens einen zweiten magnetischen Elements in distale Richtung ist das Skalenelement somit in distale Richtung verschiebbar. In dem Hohlraum ist somit distal von dem wenigstens einen zweiten magnetischen Element ein in proximale Richtung federvorgespanntes Teil des Skalenelements angeordnet.

Das Federelement, das zweite magnetische Element (oder eine magnetische Komponente desselben) und das zweite Teil des Skalenelements sind vorzugsweise in einem Hohlraum im Drehschieber bzw. Betätigungselement angeordnet. Das zweite magnetische Element und das zweite Teil des Skalenelements sind innerhalb des Hohlraums, parallel zur Längsachse des medizinischen Geräts längsverschiebbar. Durch diese Längsverschiebung ist das Federelement komprimierbar. Mit anderen Worten umfasst die Betätigungseinrichtung somit mindestens einen Hohlraum, in dem das wenigstens eine, zweite magnetische Element längsverschiebbar angeordnet ist.

In einem Ruhezustand, in dem in dem Steinfangkörbchen kein Konkrement aufgenommen ist und das Steinfangkörbchen somit vollständig in den Außentubus einziehbar ist, sind das zweite Teil und das zweite magnetische Element (oder die magnetische Komponente desselben) mittels der durch das Federelement in proximale Richtung ausgeübten Federkraft in den proximalen Endbereich, vorzugsweise an das proximale Ende des Hohlraums verschoben. Ist in dem Steinfangkörbchen dagegen ein Konkrement aufgenommen und wird der Drehschieber in seine maximal proximale Stellung verschoben, so wird das zweite magnetische Element (oder die magnetische Komponente desselben) durch das magnetische Zusammenwirken mit dem ersten magnetischen Element - welches durch seine Kopplung mit der Zug- und Schiebelitze nicht mehr in seine proximalste Stellung zurückziehbar ist - innerhalb des Hohlraums an eine distalere Position verschoben.

In einem verwandten Aspekt betrifft die Erfindung ein medizinisches Gerät, vorzugsweise zur Verwendung in einem medizinischen Endoskop, vorzugsweise ein Gerät mit magnetischer Kopplung der hierin beschriebenen Art, zum Bewegen eines medizinischen Instruments in Gerätelängsrichtung und in Drehrichtung quer um die betreffende Längsrichtung mittels einer Betätigungseinrichtung, dadurch gekennzeichnet, dass das Gerät ein Griffteil aufweist, das an seinem proximalen Ende eine Öffnung aufweist, in die ein Einführungshilfselement einführbar und in der das Einführungshilfselement klemmend arretierbar ist.

Die Erfindung betrifft auch ein medizinisches System, das ein erfindungsgemäßes medizinisches Gerät und ein Einführungshilfselement umfasst, wobei das Einführungshilfselement vorzugsweise in der proximalen Öffnung des Griffteils des medizinischen Geräts arretiert ist.

Das Einführungshilfselement kann vor dem Einführen des medizinischen Instruments in den Arbeitskanal eines Endoskops in den proximalen Eingangsstutzen des Arbeitskanals eingeführt bzw. in diesen eingesteckt werden. Es ist daher aus der Öffnung im Gerät entnehmbar. Das Einführungshilfselement ist trichterartig ausgebildet und erleichtert die Einführung des distalen Endes des medizinischen Instruments in den Eingangsstutzen. Die reversible Arretierung des Einführungshilfselements am proximalen Ende des Griffteils bietet den Vorteil, dass die sterile Umverpackung des medizinischen Geräts nur wenig am proximalen Ende geöffnet werden muss, um Zugang zu dem Einführungshilfselement zu erhalten. Zum Aufstecken des Einführungshilfselements auf den Eingangsstutzen kann das medizinische Gerät zunächst beiseitegelegt werden, ohne dass die Gefahr besteht, dass der distale Endbereich des Geräts oder Instruments unsteril wird. Anschließend kann der distale Endbereich ebenfalls aus der sterilen Umverpackung entnommen werden und zügig in den Arbeitskanal eingeführt werden.

Das Einführungshilfselement ist in der Regel zu diesem Zweck an seinem proximalen Endbereich zylindrisch ausgebildet. Der distale Endbereich des Einführungshilfselements umfasst einen in distale Richtung trichterförmig (konisch) zusammenlaufenden Abschnitt. Distal anschließend an den konischen Abschnitt kann das Einführungshilfselement einen weiteren zylindrischen Abschnitt aufweisen. Dieser Abschnitt hat einen geringeren Außenumfang als der proximale Endbereich des Einführungshilfselements. Geeignete Einführungshilfselemente sind Fachleuten bekannt.

Zur reversiblen Arretierung in der proximalen Öffnung des Handgriffs kann das Einführungshilfselement wulstartige Rippen aufweisen, die jeweils entlang eines Umfangs des Einführungshilfselements verlaufen, vorzugsweise entlang eines Umfangs im proximalen Endbereich. Entsprechend weist der Handgriff in diesen Ausführungsformen an der proximalen Öffnung Klemmelemente auf, mittels derer das Einführungshilfselement arretiert werden kann. Vorzugsweise greifen die Klemmelemente in üblicher Weise zwischen den Rippen ein und gleiten bei dem Einführen und Herausziehen des Einführungshilfselements aus der Öffnung über die Rippen hinüber. Es versteht sich, dass die Öffnung vorzugsweise mindestens teilweise formkomplementär zu dem Querschnitt des Einführungshilfselements ausgebildet ist. Vorzugsweise ist die Öffnung im Wesentlichen kreisförmig oder teilkreisförmig, wobei der Innendurchmesser des Kreises dem Außendurchmesser des Einführungshilfselements entspricht. Die Öffnung ist vorzugsweise quer zur Längsrichtung des medizinischen Geräts angeordnet

### Kurze Beschreibung der Zeichnungen

An Hand von Zeichnungen wird die Erfindung nachstehend an Ausführungsbeispielen näher erläutert.

In den Zeichnungen zeigen
- Fig. 1: eine Perspektivansicht eines medizinischen Geräts gemäß einer Ausführungsform der Erfindung in einer Größe, die von der in der Praxis verwendeten Größe verschieden sein kann,
- Fig. 2: eine Draufsicht des medizinischen Geräts gemäß Fig. 1 in einer etwas abgewandelten Ausführungsform und in einer Größe, die ebenfalls von der in der Praxis verwendeten Größe verschieden sein kann,
- Fig. 3: eine Schnittansicht der in Fig. 2 gezeigten Ausführungsform des medizinischen Geräts gemäß der Erfindung,
- Fig. 4: eine Draufsicht eines medizinischen Geräts gemäß einer noch weiteren Ausführungsform der Erfindung ebenfalls in einer Größe, die von der in der Praxis verwendeten Größe verschieden sein kann,
- Fig. 5: eine Schnittansicht der in Fig. 4 dargestellten Ausführungsform des medizinischen Geräts gemäß der Erfindung,
- Fig. 6: eine vergrößerte Schnittansicht gemäß der in Fig. 5 eingetragenen Schnittlinie I-I,
- Fig. 7: eine weitere vergrößerte Schnittansicht gemäß der in Fig. 5 eingetragenen Schnittlinie I - I,
- Fig. 8: eine Draufsicht eines medizinischen Geräts gemäß einer noch weiteren Ausführungsform der Erfindung ebenfalls in einer Größe, die von der in der Praxis verwendeten Größe verschieden sein kann,
- Fig. 9: eine Schnittansicht der in Fig. 8 dargestellten Ausführungsform des medizinischen Geräts gemäß der Erfindung,
- Fig. 10: eine Draufsicht eines medizinischen Geräts gemäß einer noch weiteren Ausführungsform der Erfindung ebenfalls in einer Größe, die von der in der Praxis verwendeten Größe verschieden sein kann,
- Fig. 11: eine Schnittansicht der in Fig. 10 dargestellten Ausführungsform des medizinischen Geräts gemäß der Erfindung,
- Fig. 12: einen vergrößerten Ausschnitt von Fig. 11,
- Fig. 13: einen anderen vergrößerten Ausschnitt von Fig. 11,
- Fig. 14: einen noch anderen vergrößerten Ausschnitt von Fig. 11,
- Fig. 15: einen noch weiteren vergrößerten Ausschnitt von Fig.11
- Fig. 16: eine Draufsicht eines medizinischen Geräts gemäß einer noch weiteren Ausführungsform der Erfindung, die ein in Fig. 17B sichtbares Skalenelement mit einem L-förmigen Querschnitt aufweist, mit dem die Länge eines im Steinfangkörbchen aufgenommenen Konkrements messbar ist, und wobei das medizinische Instrument vollständig in den Außentubus zurückgezogen ist (A: proximaler Abschnitt; B: distaler Endbereich),
- Fig. 17: eine Schnittansicht eines proximalen Teils der in Fig. 16 dargestellten Ausführungsform des medizinischen Geräts gemäß der Erfindung (A) sowie eine vergrößerte Schnittansicht des in Fig. 17A dargestellten Drehschiebers (B),
- Fig. 18: eine Draufsicht der in Fig. 16-17 dargestellten Ausführungsform, wobei ein Konkrement in dem distalen Steinfangkörbchen aufgenommen ist (A: proximaler Abschnitt; B: distaler Endbereich),
- Fig. 19: eine Schnittansicht eines proximalen Teils der in Fig. 16-18 dargestellten Ausführungsform, wobei in nicht dargestellter Weise ein Konkrement in dem distalen Steinfangkörbchen aufgenommen ist (A) sowie eine vergrößerte Schnittansicht des in Fig. 19A dargestellten Drehschiebers (B),
- Fig. 20: eine Perspektivansicht eines medizinischen Geräts gemäß der in Fig. 16 und 17 gezeigten Ausführungsform und Stellung, wobei das medizinische Instrument vollständig in den Außentubus zurückgezogen ist (A: proximaler Abschnitt; B: distaler Endbereich),
- Fig. 21: eine Perspektivansicht eines medizinischen Geräts gemäß der in Fig. 18 und 19 gezeigten Ausführungsform und Stellung, wobei ein Konkrement in dem distalen Steinfangkörbchen aufgenommen ist (A: proximaler Abschnitt; B: distaler Endbereich),
- Fig. 22: Perspektivansichten eines proximalen Teils eines medizinischen Geräts gemäß einer weiteren Ausführungsform, an deren proximalem Ende ein Fixierungshilfselement angeordnet ist, wobei das Fixierungshilfselement in (A) aus dem Abschlussgriffteil herausgenommen und in (B) in das Abschlussgriffteil eingeführt ist, und
- Fig. 23: eine seitliche Draufsicht der in Fig. 22 dargestellten Ausführungsform des erfindungsgemäßen medizinischen Geräts.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

Bevor auf die Zeichnungen näher eingegangen wird, sei zunächst angemerkt, dass in sämtlichen Zeichnungen gleiche oder einander entsprechende Einrichtungen bzw. Elemente mit gleichen Bezugszeichen bezeichnet sind.

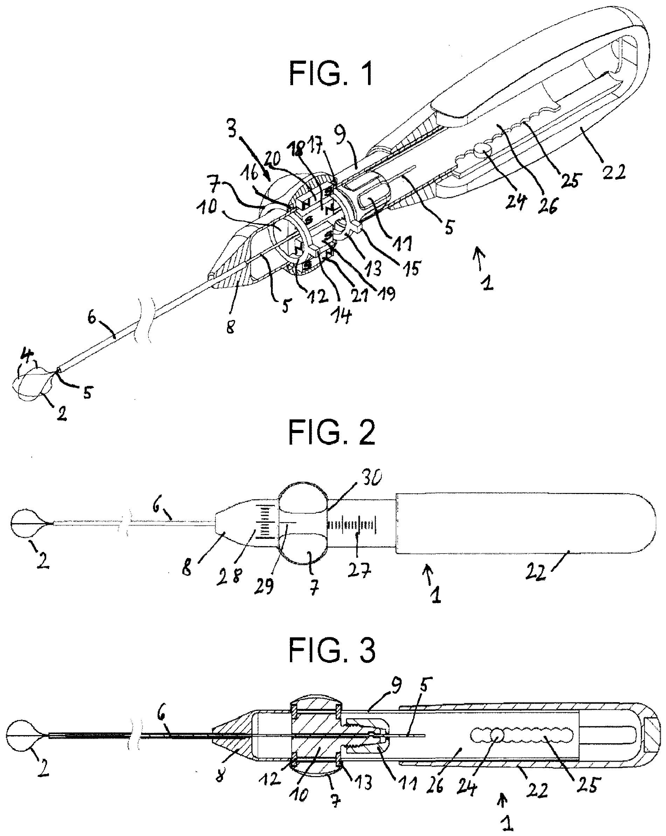

Fig. 1 zeigt eine Perspektivansicht eines medizinischen Geräts 1 gemäß einer Ausführungsform der Erfindung in einer Größe, die von der in der Praxis verwendeten Größe verschieden sein kann. Das medizinische Gerät 1 enthält ein medizinisches Instrument 2, das hier als Steinfangkörbchen 2 dargestellt ist, beispielsweise zum Einfangen von Gallensteinen oder Nierensteinen. Dieses medizinische Instrument 2 lässt sich mittels einer Betätigungseinrichtung 3 des medizinischen Geräts 1 in einer Einhandbewegung in Gerätelängsrichtung und in Drehrichtung quer um die betreffende Längsrichtung bewegen. Die Betätigungseinrichtung 3 umfasst hier, wie noch näher ersichtlich wird, einen Drehschieber 7.

Das zuvor erwähnte Steinfangkörbchen 2 besteht aus Körbchenlitzen 4, die mit einer Zug- und Schiebelitze 5 von einem Außentubus 6 aufgenommen sind, in welchem diese in dessen Längsrichtung verschiebbar ist. Durch Verschieben der Zug- und Schiebelitze 5 in Richtung zum distalen (in Fig. 1 links dargestellten) Geräteende hin können die Körbchenlitzen 4 aus dem Außentubus 6 herausgeschoben und das Steinfangkörbchen 2 geöffnet werden; durch Bewegen der Zug- und Schiebelitze 5 in Richtung zum entgegengesetzten proximalen (in Fig. 1 rechts dargestellten) Geräteende bzw. durch Einziehen der Zug- und Schiebelitze 5 in den Außentubus 6 lassen sich die Körbchenlitzen 4 mit der Zug- und Schiebelitze 5 wieder schließen und dadurch zum Beispiel Steinkonkremente in dem Steinfangkörbchen 2 aufnehmen und von diesem festhalten.

Das medizinische Gerät 1 enthält ein distal vorgesehenes Gerätespitzenteil 8, an das sich zum proximalen Geräteende hin ein Abdeckrohr 9 anschließt, welches als Verlängerungsteil des Gerätespitzenteiles 8 ausgebildet sein kann.

Von dem Gerätespitzenteil 8 ist, wie aus Fig. 1 klar hervorgeht, der Außentubus 6 des medizinischen Instruments 2 fest aufgenommen. Die innerhalb dieses Außentubus 6 geführte Zug- und Schiebelitze 5 verläuft ins Innere des erwähnten Abdeckrohres 9 und ist dort von einem Aufnahmekörper 10 fest aufgenommen, der von dem oben bereits erwähnten Drehschieber 7 umgeben ist und zusammen mit diesem die Betätigungseinrichtung 3 bildet. Diese Verhältnisse sind ganz generell in Fig. 2 und 3 veranschaulicht, die das medizinische Gerät 1 gemäß Fig. 1 mit einer etwas abgewandelten Gestaltung am proximalen Geräteende zeigen.

Die Zug - und Schiebelitze 5 ist - wie aus der Schnittansicht gemäß Fig. 3 deutlich hervorgeht - in dem Aufnahmekörper 10 durch ein auf dieses aufgeschraubtes Klemmteil 11 gegen Herausziehen aus dem und Hineinschieben in den betreffenden Aufnahmekörper 10 gesichert. Mit dem Aufnahmekörper 10 sind an dessen distalen und proximalen Enden Ringelemente 12, 13 verbunden, die mit in Fig. 1 dargestellten abstehenden Zapfen 14 bzw. 15 versehen sind, welche von einem (nicht dargestellten) Schlitz in dem Abdeckrohr 9 zur Führung aufgenommen sind. Die beiden Zapfen 12, 13 ragen mit ihren Enden dabei etwas über den Außenumfang des Abdeckrohres 9 hinaus. Jene über den Außenumfang des Abdeckrohres 9 hinausstehenden Teile der Zapfen 14, 15 sind von umlaufenden Nuten 16 bzw. 17 im Innenumfang des Drehschiebers 7 aufgenommen. Durch diese mechanische Koppelung kann der Drehschieber 7 relativ zu dem Aufnahmekörper 10 quer zur Gerätelängsrichtung gedreht und zusammen mit dem Aufnahmekörper 10 in der Gerätelängsrichtung verschoben werden.

Um die zuvor erwähnte Drehbewegung des Drehschiebers in eine Drehbewegung der Zug- und Schiebelitze 5 und damit in eine entsprechende Drehbewegung des medizinischen Steinfangkörbchens 2 umzusetzen, ist gemäß der Erfindung der die Zug- und Schiebelitze 5 festhaltende Aufnahmekörper 10 mit wenigstens einem ersten magnetischen Element - gemäß Fig. 1 mit zwei diametral gegenüber liegenden ersten magnetischen Elementen 18,19 versehen. Diese magnetischen Elemente 18,19 sind hier durch einzelne stabförmige Permanentmagneten gebildet, die an dem Aufnahmekörper 10 fest angebracht sind, beispielsweise durch Ankleben. Die Ausrichtung der Magnetpole - Nordpol (N) und Südpol(S) - verläuft hier in Gerätelängsrichtung.

Den beiden Permanentmagneten 18, 19 in geringem Abstand gegenüber liegend befindet sich wenigstens ein zweites magnetisches Element - das hier durch zwei entsprechende stabförmige Permanentmagneten 20 bzw. 21 gebildet ist, die innerhalb des Drehschiebers 7 an dem Aufnahmekörper 10 angebracht sind, beispielsweise ebenfalls durch Ankleben. Die Ausrichtung der Magnetpole - Nordpol (N) und Südpol(S) - der Permanentmagneten 20, 21 verläuft hier in Gerätelängsrichtung - entgegengesetzt zur Ausrichtung der Magnetpole - Nordpol (N) und Südpol(S) - der Permanentmagneten 18 bzw. 19. Dadurch ziehen sich die Permanentmagneten 18 und 20 sowie die Permanentmagneten 19 und 21 jeweils einander an. Es sei hier angemerkt, dass die Permanentmagneten 18 und 20 sowie 19 und 21 auch jeweils magnetisch gleichpolig zueinander ausgerichtet sein könnten, so dass jeweils Nordpole (N) und Südpole (S) einander gegenüber liegend vorgesehen sind. Die Drehbewegung des medizinischen Geräts bzw. Steinfangkörbchens 2 durch Drehen des Drehschiebers 7 erfolgt hier also gemäß Fig. 1 durch magnetische Koppelung der Permanentmagneten 18 und 20 sowie 19 und 21 während die Längsverschiebung des medizinischen Geräts bzw. Steinfangkörbchens 2 durch mechanische Koppelung des Drehschiebers 7 mit dem erwähnten Aufnahmekörper 10 erfolgt.

Zum proximalen (in Fig. 1 rechts dargestellten) Geräteende schließt sich an das Abdeckrohr 9 ein Abschlussgriffteil 22 an, das bauchig ausgebildet ist und das auf das Abdeckrohr 9 verschiebbar aufgesteckt sein kann. Dieses Abschlussgriffteil 22 trägt hier in seinem Innern eine Rastrolle 24, die in Rastöffnungen 25 einer Rastelementenleiste 26 anzuliegen vermag, welche hier als Teil des Abdeckrohres 9 gebildet ist.

In Fig. 2 ist dargestellt, dass das medizinische Gerät 1 mit einer Skaleneinrichtung versehen ist, die hier aus zwei Skalen 27 und 28 besteht, welche Einteilungen beispielsweise im Millimeter/Zoll bzw. in Winkelgraden tragen und zusätzlich mit Maßangaben versehen sein können. Die Skala 27 für Längenangaben befindet sich auf der Außenseite des Abdeckrohres 9; als Bezugselement bei der Messung des Ausfahrens des medizinischen Instruments bzw. Steinfangkörbchens aus dem medizinischen Gerät 1 kann hier die in Fig. 2 rechts dargestellte Außenkante 30 des Drehschiebers 7 herangezogen werden. Mittels der auf dem distalen Gerätespitzenteil 4 aufgebrachten Skala 28, die zum Erfassen von Winkelgraden dient, kann die Drehung des Drehschiebers 7 und damit des medizinischen Instruments bzw. Steinfangkörbchens erfasst werden. Für dieses Erfassen ist dieser Skala 28 ein auf der Außenseite des Drehschiebers 7 nahe der Skala aufgebrachter Zeiger 29 zugehörig.

Die in Fig. 3 dargestellte Schnittansicht des in Fig. 2 gezeigten medizinischen Geräts 1 lässt erkennen, dass hier im Unterschied zu der in Fig. 1 gezeigten Ausführungsform das Abschlussgriffteil 22 zylinderförmig ausgebildet ist. Wie in Fig. 1 sind auch hier die Rastelementenleiste 26 mit den Rastöffnungen 25 und der Rastrolle 24 vorgesehen.

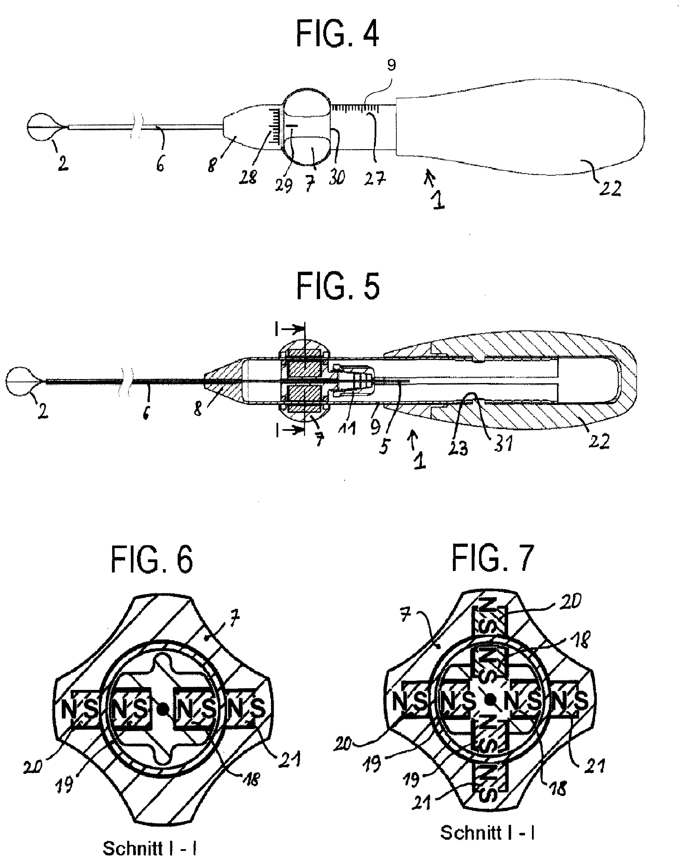

In Fig. 4 und 5 ist ein medizinisches Gerät 1 gemäß einer noch weiteren Ausführungsform der Erfindung in Draufsicht bzw. in einer Schnittansicht dargestellt. Hier ist das bauchig ausgebildete Abschlussgriffteil 22 auf dem Abdeckrohr 9 durch eine Rastverbindung festgehalten, die aus einer Rastnase 31 an dem Abschlussgriffteil 22 und einer dazu passenden Arretierungsöffnung 23 in dem Abdeckrohr 9 besteht.

Fig. 6 und Fig. 7 zeigen vergrößerte Schnittansichten längs der in Fig. 5 eingetragenen Schnittlinie I - I. Dabei zeigt Fig. 6 erste magnetische Elemente 18, 19 und zweite magnetische Elemente 20, 21 jeweils als stabförmige Permanentmagneten auf diametral gegenüber liegenden Seiten des Aufnahmekörpers 10 bzw. des Drehschiebers 7. Die Polung der Permanentmagneten 18, 21 und 19, 20 ist dabei so, dass sich deren Gegenpole (Nordpole N und Südpole S) einander gegenüber liegen und sich die betreffenden Permanentmagneten somit anziehen. Fig. 7 zeigt eine gekreuzte Anordnung von zwei zueinander um 90° versetzten Sätzen von Permanentmagneten 18 bis 21 mit entsprechender Polung (N und S).

An dieser Stelle sei angemerkt, dass die erwähnten Permanentmagneten 18, 21 und 19, 20 prinzipiell auch anders gepolt sein können als in Fig. 6 und 7 dargestellt, das heißt jeweils mit gleichen Polen (entweder N-Polen oder S-Polen) einander gegenüber liegend angeordnet sein können.

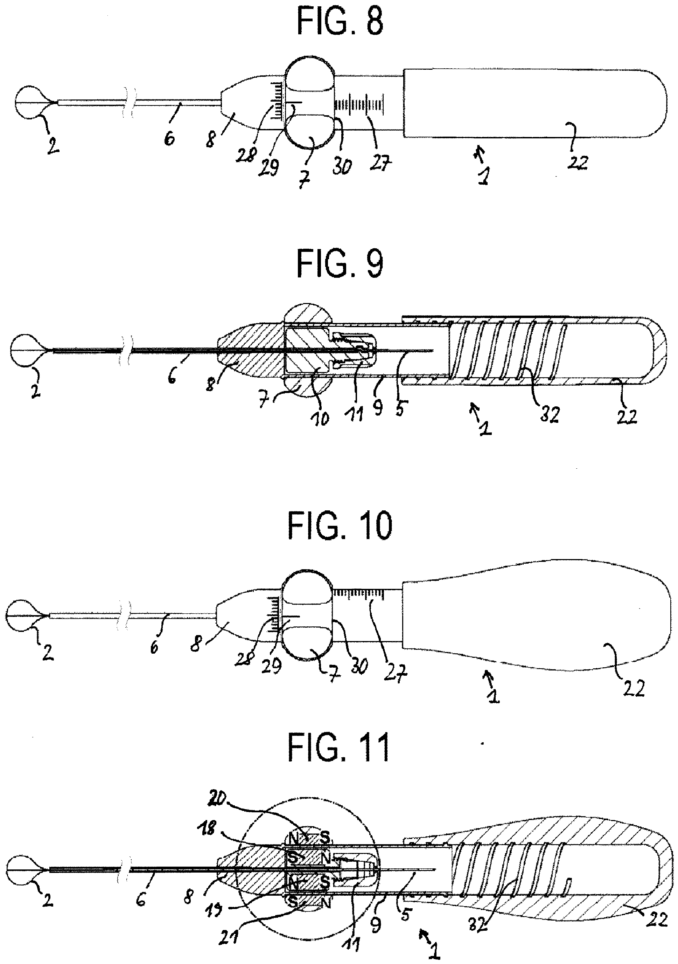

In Fig. 8, 9, 10 und 11 sind weitere Modifikationen von Ausführungsformen des medizinischen Geräts 1 gemäß der Erfindung dargestellt. Diese Modifikationen unterscheiden sich von den zuvor betrachteten Ausführungsformen des medizinischen Geräts 1 gemäß der Erfindung im Wesentlichen dadurch, dass das jeweilige Abschlussgriffteil 22 hier mittels eines Schraubgewindes 32 auf das Abdeckrohr 9 aufgeschraubt ist. Dadurch lässt sich mit dem für eine Handauflage während der Einhand-Gerätebedienung nutzbaren proximalen Gerätebereich eine für den jeweiligen Benutzer angenehme Länge sowie eine gewünschte Öffnungslänge für das Steinfangkörbchen 2 einfach einstellen.

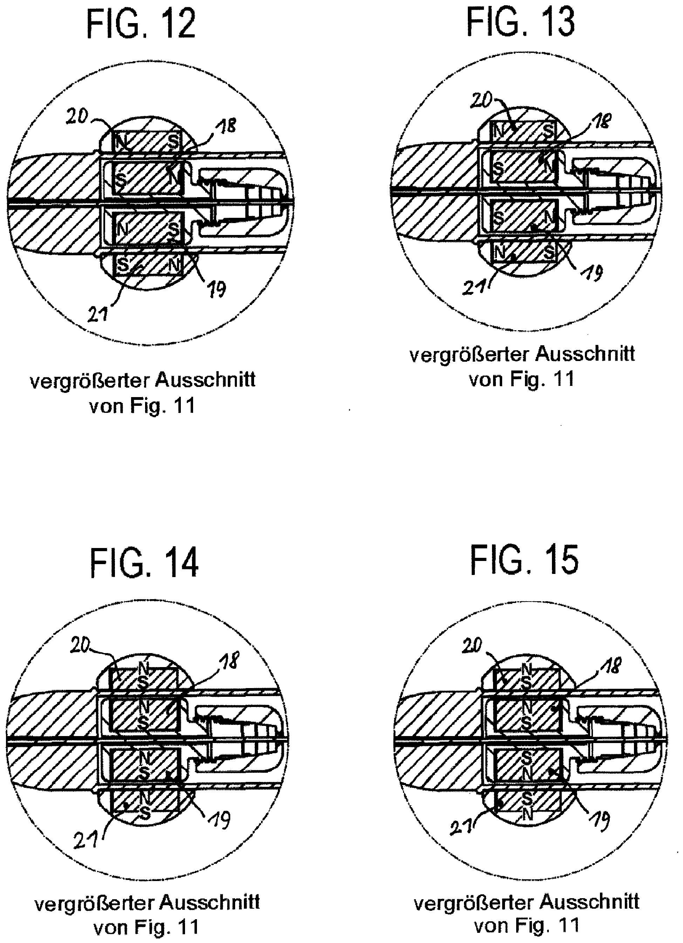

Fig. 12, 13, 14 und 15 zeigen vergrößerte Ausschnitte eines in Fig. 11 durch einen Strich-Punkt-Kreis umrahmten Bereichs - das ist der den Drehschieber 7 und den Aufnahmekörper 10 umfassende Betätigungsbereich 3 des medizinischen Geräts 1.

Dabei zeigen Fig. 12 und Fig. 13 den Einsatz von stabförmigen Permanentmagneten 18, 19, 20 und 21, deren Polung (Nordpole N und Südpole S) jeweils in Längsrichtung des medizinischen Geräts 1 ausgerichtet sind. In Fig. 12 ziehen sich die einander gegenüberliegenden Permanentmagneten 18, 20 und 19, 21 mit ihren Gegenpolen (N - S) einander an. Demgegenüber stoßen sich in Fig. 13 die einander gegenüber liegenden Permanentmagneten 18, 20 und 19, 21 mit ihren Gegenpolen (N - S) voneinander ab.

Fig. 14 und Fig. 15 veranschaulichen den in Fig. 12 und Fig. 13 bezüglich der Permanentmagnete 18 bis 21 dargestellten Verhältnisse entsprechende Verhältnisse für Permanent-Rundmagneten 18 bis 21, deren Gegenpole (Nord - Süd) jeweils quer zu der Gerätelängsrichtung ausgerichtet sind. Dabei zeigt Fig. 14, dass sich die einander gegenüber liegenden Permanent-Rundmagneten 18, 20 und 19, 21 mit ihren Gegenpolen (N - S) einander anziehen. Demgegenüber stoßen sich in Fig. 15 die einander gegenüber liegenden Permanent-Rundmagneten 18, 20 und 19, 21 mit ihren Gegenpolen (N - S) voneinander ab.

Abschließend sei noch angemerkt, dass die Erfindung auch mit anderen medizinischen Instrumenten 2 als dem beschriebenen Steinfangkörbchen realisiert sein kann. So können als medizinische Instrumente beispielsweise Zangen, wie endoskopisch einsetzbare Zangen, Schlingen, wie Polypektomieschlingen, oder medizinische oder chirurgische Handhabungswerkzeuge verwendet werden.

Die zumindest einen magnetischen Elemente 18, 19, 20, 21 sind zuvor als jeweils zwei oder vier magnetische Elemente enthaltend beschrieben worden. Es sei jedoch angemerkt, dass die betreffenden magnetischen Elemente auch in größerer Anzahl vorgesehen sein können, und zwar vorzugsweise stets paarweise ein erstes magnetisches Element und ein zweites magnetisches Element. Dabei können, wie eingangs bereits erwähnt, einige, mehrere oder sämtliche der betreffenden magnetischen Elemente durch elektromagnetische Elemente gebildet sein.

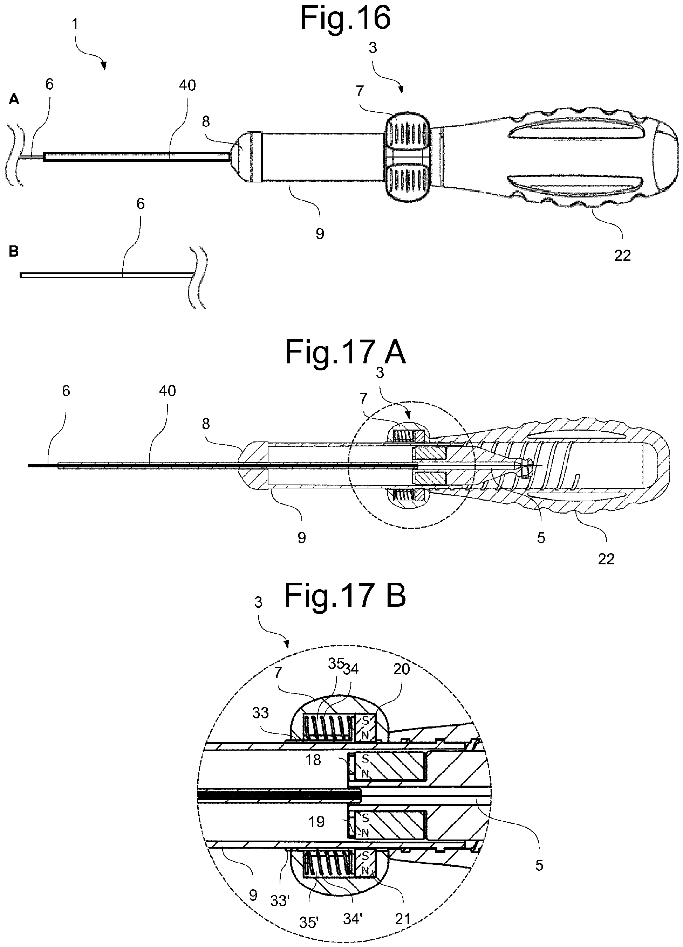

Die Fig. 16 bis 21 zeigen dieselbe Ausführungsform eines erfindungsgemäßen medizinischen Geräts 1, in der die Betätigungseinrichtung 3 einen Drehschieber 7 und ein Skalenelement 33 mit einem L-förmigen Querschnitt aufweist. Im Übrigen ähnelt die hier dargestellte Ausführungsform in vielen Elementen der in den Fig. 10 und 11 dargestellten Ausführungsform. Die dort bereits beschriebenen Elemente sollen daher hier nicht ausführlich nochmals beschrieben werden.

Es ist erkennbar, dass sowohl der Drehschieber 7 als auch das Abschlussgriffteil 22 Riffelungen aufweisen, die ein besonders sicheres Halten und Bedienen der Elemente ermöglichen.

In den Fig. 16 bis 21 sind die ersten magnetischen Elemente 18, 19 jeweils durch magnetisches Zusammenwirken an ihren Gegenpolen in den zweiten magnetischen Elementen 20, 21 ausgerichtet.

In den Schnittansichten der Fig. 17A und B ist erkennbar, dass der Drehschieber 7 des medizinischen Geräts 1 zwei Hohlräume 35, 35' aufweist. Die Hohlräume 35, 35' sind radial in Richtung des Abdeckrohres 9 offen. Sie erstrecken sich länglich parallel zu der Längsachse des medizinischen Geräts 1 und damit auch parallel zu dem Abdeckrohr 9. Innerhalb der Hohlräume 35, 35' sind jeweils ein Federelement 34 bzw. 34', ein zweites magnetisches Element 20 bzw. 21 und ein Teil eines Skalenelements 33 bzw. 33' angeordnet. Dabei ist das Federelement 34 bzw. 34' distal von dem Teil eines Skalenelements 33 bzw. 33 und das Teil eines Skalenelements 33 bzw. 33' distal von dem zweiten magnetischen Element 20 bzw. 21 angeordnet. Die Länge der Hohlräume 35, 35' entspricht vorzugsweise jeweils der kombinierten Länge des zweiten magnetischen Elements 20 bzw. 21, des Federelements 34 bzw. 34' und des Teils des Skalenelements 33 bzw. 33', wobei das Federelement 34 bzw. 34' nicht vollständig komprimiert ist. Während das Federelement 34 bzw. 34' in dieser Position nicht vollständig entspannt sein muss, so ist es doch bevorzugt, dass die Federelemente 34, 34' in dieser Ruhestellung nahezu oder ganz entspannt sind, d.h. im Wesentlichen vollständig entspannt. Hierdurch wird sichergestellt, dass die Federelemente 34, 34' in distale Richtung komprimierbar sind.

In der in Fig. 16, 17 und 20 dargestellten Stellung ist das medizinische Instrument 2 vollständig in proximale Richtung in den Außentubus 6 des medizinischen Geräts 1 zurückgezogen. Diese Stellung wird hierin auch als "Ruhestellung" oder "Ruheposition" bezeichnet. Das vollständige Zurückziehen des medizinischen Instruments 2 in den Außentubus 6 ist nur möglich, wenn zwischen den Körbchenlitzen 4 kein Konkrement 42 aufgenommen ist. In dieser Stellung kann das medizinische Gerät 1 beispielsweise in eine Körperöffnung des Patienten eingeführt und an den Eingriffsort gebracht werden.

Um ein Abknicken des Außentubus 6 hinter dem Gerätespitzenteil 8 zu verhindern, weist das medizinische Gerät 1 in den Fig. 16 bis 23 einen Knickschutztubus 40 auf, der sich unmittelbar distal des Gerätespitzenteils 8 um den Außentubus 6 herumlegt und diesen stabilisiert. Der Knickschutztubus 40 ist ein schlauchförmiges, flexibles Element in dessen Inneren der Außentubus 40 verläuft. Der Knickschutztubus 40 ist deutlich kürzer als der Außentubus 40.

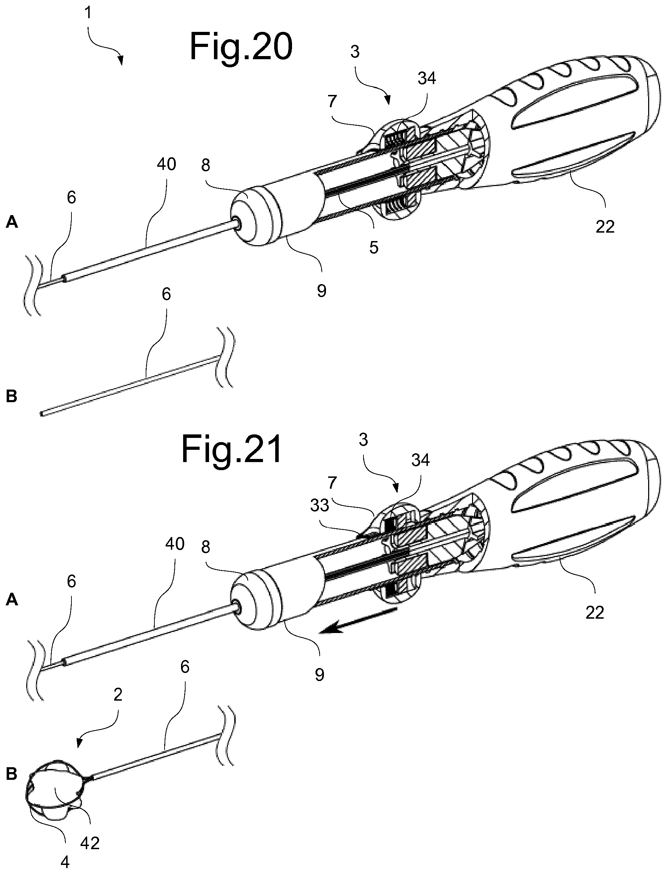

Es ist in den Fig. 17 und 20 erkennbar, dass die Federelemente 34, 34' im Wesentlichen entspannt sind und eine Federkraft auf das zweite Teil des Skalenelements 33, 33' in proximale Richtung ausüben. Das Skalenelement 33 bzw. 33' weist einen L-förmigen Querschnitt auf und ist somit aus einem ersten Teil aufgebaut, das sich parallel zur Längsachse des medizinischen Geräts 1 erstreckt, und einem zweiten Teil, das sich von dem proximalen Ende des ersten Teils aus orthogonal von dem Abdeckrohr 9 radial in den Hohlraum 35 bzw. 35' erstreckt. In dem dargestellten Ausführungsbeispiel ist das Skalenelement 33, 33' aus einem Stück gefertigt, wobei zwischen dem ersten und dem zweiten Teil des Skalenelements 33, 33' ein 90° Winkel liegt. Die Federkraft des Federelements 34, 34' drückt das zweite Teil des Skalenelements 33, 33' gegen das zweite magnetische Element 20 bzw. 21.

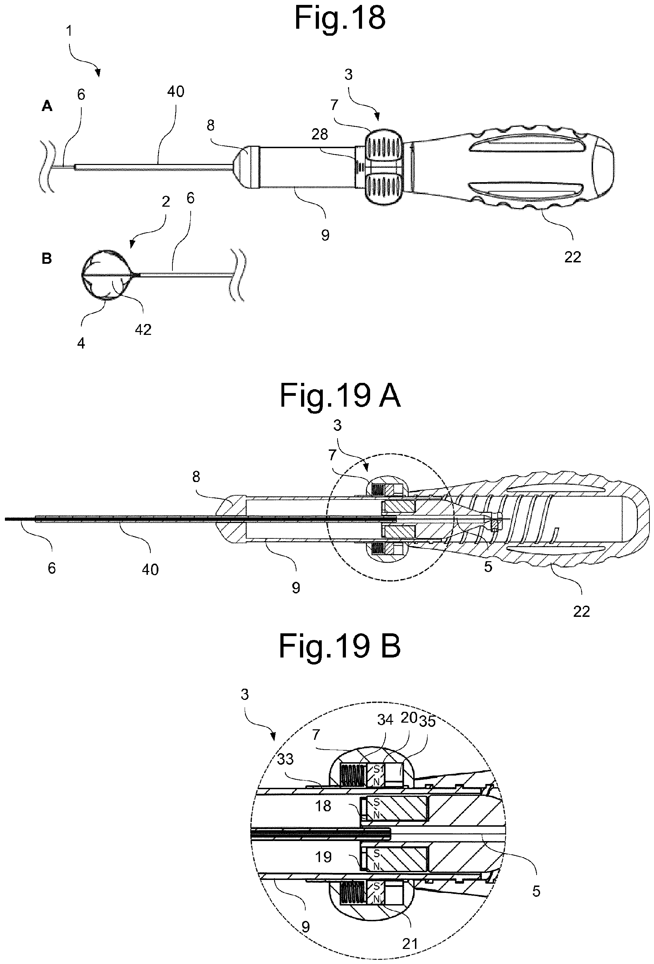

In den Fig. 18, 19 und 21 ist eine Stellung gezeigt, in der das Steinfangkörbchen 2 distal aus dem Außentubus 6 herausgeschoben ist und ein Konkrement 42, z, B, einen Nierenstein, zwischen den Körbchenlitzen 4 aufgenommen hat. Die Zug- und Schiebelitze 5 ist dabei soweit wie möglich in den Außentubus 6 zurückgezogen, d. h. so weit, dass sich die Körbchenlitzen 4 auf im Wesentlichen ihrer ganzen Länge dicht um das Konkrement 42 herumgelegt haben. Der Drehschieber 7 wurde nach dem Erreichen dieser Stellung noch weiter in proximale Richtung bewegt, sodass sich durch das magnetische Zusammenwirken der ersten und zweiten magnetischen Elemente 18, 19, 20, 21 und unter Kompression des Federelements 34, 34' das zweite Teil des Skalenelements 33, 33' in distale Richtung verschob. Durch die Verschiebung des Skalenelements 33, 33' in distale Richtung ist der distale Abschnitt des ersten Teils des Skalenelements 33, 33' zwischen dem Drehschieber 7 und dem Abdeckrohr 9 hervorgeschoben, sodass die auf dem Skalenelement 33, 33' angeordnete Skala für den Benutzer des Elements ablesbar ist.

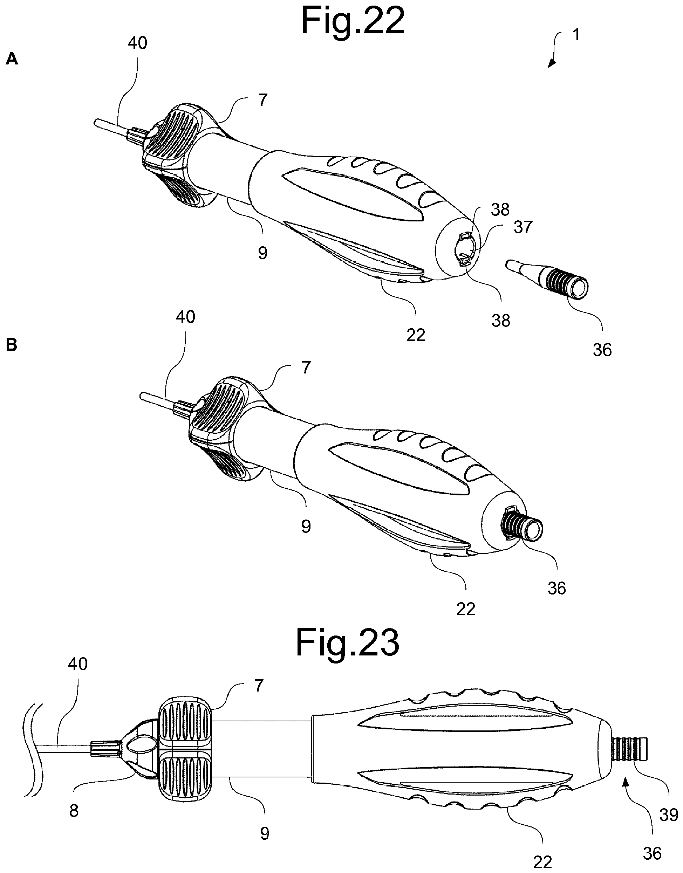

Fig. 22 und 23 zeigen Perspektivansichten Fig. 22A und B und eine Seitenansicht Fig. 23 eines medizinischen Geräts 1 gemäß einer weiteren erfindungsgemäßen Ausführungsform, an deren proximalem Ende ein Einführungshilfselement 36 angeordnet ist. Das medizinische Gerät 1 weist distal des Abschlussgriffteils 22 einen Außentubus 6 auf, in dem die hier nicht dargestellte Zug- und Schiebelitze 5 verläuft. Das Abschlussgriffteil 22 umfasst einen Drehschieber 7 der erfindungsgemäßen Art, der ein Abdeckrohr 9 umschließt, das sich proximal von dem Drehschieber 7 im Abschlussgriffteil 22 fortsetzt.

An seinem proximalen Ende weist das medizinische Gerät 22 eine Öffnung 37, in die ein Einführungshilfselement 36 einführbar ist. Wie in Fig. 23 erkennbar ist, weist das Einführungshilfselement 36 einen zylindrischen proximalen Bereich auf, auf dessen Außenumfang umlaufende Rippen 39 angeordnet sind. Beim Einführen des Einführungshilfselements 36 in die form- und größenkomplementäre Öffnung 37 greifen an der Öffnung 37 angeordnete Klemmelemente 38 in die Zwischenräume zwischen den Rippen 39 ein und gleiten über die Rippen 39 hinweg. Das Einführungshilfselement 36 weist, wie in Fig. 22 erkennbar ist, einen kurzen zylindrischen distalen Abschnitt sowie einen proximal davon angeordneten Abschnitt auf, der zylindrisch nach distal zusammenläuft. In Fig. 22 zeigt Teil A der Abbildung eine Stellung, in der das Einführungshilfselement 36 aus dem Abschlussgriffteil 22 entnommen ist, und Teil B eine Stellung, in der das Einführungshilfselement 36 in dem Abschlussgriffteil 22 arretiert ist.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

### Bezugszeichenliste

- 1: medizinisches Gerät
- 2: medizinisches Instrument, Steinfangkörbchen
- 3: Betätigungseinrichtung
- 4: Körbchenlitzen
- 5: Zug- und Schiebelitze
- 6: Außentubus
- 7: Drehschieber
- 8: Gerätespitzenteil
- 9: Abdeckrohr
- 10: Instrumenten-Aufnahmekörper, Aufnahmekörper
- 11: Klemmteil
- 12: Ringelement
- 13: Ringelement
- 14: Zapfen
- 15: Zapfen
- 16: Nut
- 17: Nut
- 18: erstes magnetisches Element, Permanentmagnet
- 19: erstes magnetisches Element, Permanentmagnet
- 20: zweites magnetisches Element, Permanentmagnet
- 21: zweites magnetisches Element, Permanentmagnet
- 22: Abschlussgriffteil
- 23: Arretierungsöffnung
- 24: Rastrolle
- 25: Rastöffnungen
- 26: Rastelementenleiste
- 27: Skala
- 28: Skala
- 29: Zeiger
- 30: Außenkante
- 31: Rastnase
- 32: Schraubgewinde
- 33, 33': Skalenelement
- 34, 34': Federelement
- 35, 35': Hohlraum
- 36: Einführungshilfselement
- 37: Proximale Öffnung
- 38: Klemmelement
- 39: Rippe
- 40: Knickschutztubus
- 42: Konkrement

## Patentansprüche

1. Medizinisches Gerät (1), vorzugsweise Gerät zur Verwendung in einem medizinischen Endoskop, zum Bewegen eines medizinischen Instruments (2) in Gerätelängsrichtung und in Drehrichtung quer um die Gerätelängsrichtung mittels einer Betätigungseinrichtung (3), wobei das medizinische Instrument (2) wenigstens ein erstes magnetisches Element (18, 19) enthält oder mit diesem verbunden und die Betätigungseinrichtung (3) wenigstens ein zweites magnetisches Element (20, 21) enthält oder mit diesem verbunden ist, **dadurch gekennzeichnet, dass** das wenigstens eine zweite magnetische Element (20, 21) von einem Drehschieber (7) der Betätigungseinrichung (3) aufgenommen ist, der zusammen mit dem wenigstens einen zweiten magnetischen Element (20, 21) um das wenigstens eine erste magnetische Element (18, 19) sowohl in der Gerätelängsrichtung als auch in Drehrichtung quer dazu verschiebbar ist,
- und dass die Betätigungseinrichtung (3) zusammen mit dem zumindest einen zweiten magnetischen Element (20, 21) entweder mechanisch gekoppelt ist und das zumindest eine erste magnetische Element (18, 19) durch magnetische Koppelung lediglich in der genannten Drehrichtung zu bewegen gestattet oder ohne mechanische Koppelung das zumindest eine erste magnetische Element (18, 19) sowohl in der genannten Gerätelängsrichtung als auch in der betreffenden Drehrichtung durch magnetische Koppelung zu bewegen gestattet.

2. Medizinisches Gerät nach Anspruch 1, **dadurch gekennzeichnet, dass** das medizinische Gerät (1) einen langgestreckten Außentubus (6) aufweist, den das medizinische Instrument (2) durchläuft.

3. Medizinisches Gerät nach einem der Ansprüche 1 und 2, **dadurch gekennzeichnet, dass** das medizinische Instrument (2) in dem medizinischen Gerät (1) längsverschiebbar und um seine Längsachse drehbar gelagert ist.

4. Medizinisches Gerät nach einem der vorangehenden Ansprüche, **dadurch gekennzeichnet, dass** der Betätigungseinrichtung (3) eine Skaleneinrichtung (27, 30, 28, 29) zugeordnet ist, die das Ausmaß zumindest einer Bewegung des medizinischen Instruments (2) in Gerätelängsrichtung und/oder in der Drehrichtung quer dazu zu ermitteln bzw. anzuzeigen gestattet.

5. Medizinisches Gerät nach einem der vorangehenden Ansprüche, **umfassend** folgende Merkmale:
- das medizinische Gerät (1) weist ein seine Handhabung ermöglichendes oder zumindest erleichterndes proximales Ende und ein das medizinische Instrument (2) enthaltendes distales Ende auf,
- das medizinische Instrument (2) weist ein von dem distalen Ende des Geräts (1) abstehendes oder aus diesem herausführbares Arbeitsende auf und ist mit seinem an dieses Arbeitsende zum proximalen Ende des Gerätes (1) hin sich anschließenden Bereich von einem Instrumenten-Aufnahmekörper (10) sowohl in der Gerätelängsrichtung als auch in Drehrichtung quer dazu verschiebbar,
- in dem Instrumenten-Aufnahmekörper (10) ist das wenigstens eine erste magnetische Element (18, 19) mit dem medizinischen Instrument (2) gekoppelt
- und das wenigstens eine erste magnetische Element (18, 19) ist von dem wenigstens einen zweiten magnetischen Element (20, 21) umgeben, welches in Bezug auf das wenigstens eine erste magnetische Element (18, 19) in Gerätelängsrichtung und in Drehrichtung quer dazu bewegbar ist und welches mit dem wenigstens einen ersten magnetischen Element (18, 19) magnetisch zusammenwirkt.

6. Medizinisches Gerät nach Anspruch 1, **dadurch gekennzeichnet, dass** der Drehschieber (7) zwischen dem Aufnahmekörper (10) und einem in der Gerätelängsrichtung proximal vorgesehenen, als Handauflagekörper nutzbaren Verlängerungsteil (22) verschieb- und drehbar ist, der in dem Aufnahmekörper (10) aufnehmbar ist.

7. Medizinisches Gerät nach einem der vorangehenden Ansprüche, **dadurch gekennzeichnet, dass** das wenigstens eine erste magnetische Element (18, 19) und das wenigstens eine zweite magnetische Element (20, 21) so angeordnet sind, dass sie mit magnetischen Gegenpolen (Nordpole N und Südpole S) zusammen wirken.

8. Medizinisches Gerät nach einem der vorangehenden Ansprüche, **dadurch gekennzeichnet, dass** die jeweiligen magnetischen Elemente (18, 19, 20, 21) durch Stabmagneten gebildet sind, deren Gegenpole (Nordpole N - Südpole S) jeweils in der Gerätelängsrichtung ausgerichtet sind.

9. Medizinisches Gerät nach einem der vorangehenden Ansprüche, **dadurch gekennzeichnet, dass** die jeweiligen magnetischen Elemente (18, 19, 20, 21) durch Rundmagneten gebildet sind, deren Gegenpole (Nordpole N - Südpole S) jeweils quer zu der Gerätelängsrichtung ausgerichtet sind.

10. Medizinisches Gerät nach einem der vorangehenden Ansprüche, **dadurch gekennzeichnet, dass** die jeweiligen magnetischen Elemente (18, 19, 20, 21) Permanentmagneten sind.

11. Medizinisches Gerät nach einem der vorangehenden Ansprüche, **dadurch gekennzeichnet, dass** das medizinische Instrument (2) ein endoskopisch einsetzbares medizinisches Instrument, ein medizinisches Steinfangkörbchen oder eine medizinische Schlinge, wie eine Polypektomieschlinge, ist.

12. Medizinisches Gerät nach einem der vorangehenden Ansprüche, **dadurch gekennzeichnet, dass** die Betätigungseinrichtung (3) mindestens einen Hohlraum (35, 35') umfasst, in dem das wenigstens eine zweite magnetische Element (20, 21) längsverschiebbar angeordnet ist.

13. Medizinisches Gerät nach Anspruch 12, **dadurch gekennzeichnet, dass** mittels einer Längsverschiebung des wenigstens einen zweiten magnetischen Elements (20, 21) in distale Richtung ein Skalenelement (33, 33') in distale Richtung verschiebbar ist.

14. Medizinisches Gerät nach einem der Ansprüche 12 und 13, **dadurch gekennzeichnet, dass** in dem Hohlraum (35, 35') distal von dem wenigstens einen zweiten magnetischen Element (20, 21) ein in proximale Richtung federvorgespanntes Teil des Skalenelements (33, 33') angeordnet ist.

## Claims

1. Medical device (1), preferably device for use in a medical endoscope, for moving a medical instrument (2) in the device longitudinal direction and in the direction of rotation transversely around the device longitudinal direction by means of an actuating device (3), the medical instrument (2) containing or being connected to at least one first magnetic element (18, 19) and
the actuating device (3) containing or being connected to at least one second magnetic element (20, 21), **characterised in that** the at least one second magnetic element (20, 21) is received by a rotary slide (7) of the actuating device (3), which rotary slide, together with the at least one second magnetic element (20, 21), can be displaced around the at least one first magnetic element (18, 19) both in the device longitudinal direction and in the direction of rotation transversely thereto,
- and **in that** the actuating device (3), together with the at least one second magnetic element (20, 21), is either mechanically coupled and allows the at least one first magnetic element (18, 19) to be moved only in said direction of rotation by magnetic coupling or, without mechanical coupling, allows the at least one first magnetic element (18, 19) to be moved both in said device longitudinal direction and in the relevant direction of rotation by magnetic coupling.

2. Medical device according to claim 1, **characterised in that** the medical device (1) has an elongate outer tube (6) through which the medical instrument (2) passes.

3. Medical device according to either claim 1 or claim 2, **characterised in that** the medical instrument (2) is mounted in the medical device (1) so as to be longitudinally displaceable and rotatable about its longitudinal axis.

4. Medical device according to any of the preceding claims, **characterised in that** the actuating device (3) is assigned a scale device (27, 30, 28, 29) which allows the extent of at least one movement of the medical instrument (2) in the device longitudinal direction and/or in the direction of rotation transversely to be determined and displayed.

5. Medical device according to any of the preceding claims, comprising the following features:
- the medical device (1) has a proximal end that allows or at least facilitates handling and a distal end containing the medical instrument (2),
- the medical instrument (2) has a working end that protrudes from the distal end of the device (1) or can be guided out of said distal end and, with its region adjoining this working end towards the proximal end of the device (1), can be moved both in the device longitudinal direction and in the direction of rotation transversely thereto by means of an instrument receiving body (10),
- in the instrument receiving body (10), the at least one first magnetic element (18, 19) is coupled to the medical instrument (2)
- and the at least one first magnetic element (18, 19) is surrounded by the at least one second magnetic element (20, 21), which can be moved with respect to the at least one first magnetic element (18, 19) in the device longitudinal direction and in the direction of rotation transversely thereto and which interacts magnetically with the at least one first magnetic element (18, 19).

6. Medical device according to claim 1, **characterised in that** the rotary slide (7) can be displaced and rotated between the receiving body (10) and an extension part (22) which is provided proximally in the device longitudinal direction, can be used as a hand support body and can be received in the receiving body (10).

7. Medical device according to any of the preceding claims, **characterised in that** the at least one first magnetic element (18, 19) and the at least one second magnetic element (20, 21) are arranged in such a way that they interact via opposite magnetic poles (north poles N and south poles S).

8. Medical device according to any of the preceding claims, **characterised in that** the magnetic elements (18, 19, 20, 21) are formed by bar magnets of which the opposite poles (north poles N - south poles S) are each aligned in the device longitudinal direction.

9. Medical device according to any of the preceding claims, **characterised in that** the magnetic elements (18, 19, 20, 21) are formed by round magnets of which the opposite poles (north poles N - south poles S) are each aligned transversely to the device longitudinal direction.

10. Medical device according to any of the preceding claims, **characterised in that** the magnetic elements (18, 19, 20, 21) are permanent magnets.

11. Medical device according to any of the preceding claims, **characterised in that** the medical instrument (2) is a medical instrument which can be used endoscopically, a medical stone retrieval basket or a medical snare such as a polypectomy snare.

12. Medical device according to any of the preceding claims, **characterised in that** the actuating device (3) comprises at least one cavity (35, 35') in which the at least one second magnetic element (20, 21) is arranged so as to be longitudinally displaceable.

13. Medical device according to claim 12, **characterised in that** a scale element (33, 33') can be displaced in the distal direction by means of a longitudinal displacement of the at least one second magnetic element (20, 21) in the distal direction.

14. Medical device according to either claim 12 or claim 13, **characterised in that** a part of the scale element (33, 33') that is spring-preloaded in the proximal direction is arranged in the cavity (35, 35') distally from the at least one second magnetic element (20, 21).

## Revendications

1. Appareil médical (1), de préférence pour une utilisation dans un endoscope médical, pour mouvoir un instrument médical (2) en sens longitudinal de l'appareil et en rotation transversale par rapport à l'axe longitudinal de l'appareil au moyen d'un dispositif d'actionnement (3), l'instrument médical (2) comprenant au moins un premier élément magnétique (18, 19) ou y étant associé et le dispositif d'actionnement (3) comprenant au moins un second élément magnétique (20, 21) ou y étant associé, **caractérisé en ce que**
- le (les) second(s) élément(s) magnétique(s) (20, 21) est (sont) logé(s) dans un curseur rotatif (7) du dispositif d'actionnement (3), lequel curseur est déplaçable conjointement avec le (les) second(s) élément(s) magnétique(s) (20, 21) autour du (des) premier(s) élément(s) magnétique(s) (18, 19) aussi bien en sens longitudinal de l'appareil qu'en rotation transversale par rapport au sens longitudinal,
- et **en ce que** le dispositif d'actionnement (3) est soit mécaniquement couplé au (aux) second(s) élément(s) magnétique(s) (20, 21) et ne permet, par accouplement magnétique, qu'un mouvement de rotation du (des) premier(s) éléments) magnétique(s) (18, 19) dans la direction indiquée, soit qu'il permet par accouplement magnétique et sans accouplement mécanique aussi bien un déplacement du (des) premier(s) élément(s) magnétique(s) (18, 19) dans le sens longitudinal de l'appareil qu'une rotation dans le sens indiqué.

2. Appareil médical selon la revendication 1, **caractérisé en ce que** l'appareil médical (1) présente un tube extérieur (6) allongé que traverse l'instrument médical.

3. Appareil médical selon l'une des revendications 1 ou 2, **caractérisé en ce que** l'instrument médical (2) est logé dans l'appareil médical (1) de façon à pouvoir coulisser longitudinalement et pivoter autour de son axe longitudinal.

4. Appareil médical selon l'une des revendications précédentes, **caractérisé en ce qu'**un dispositif gradué (27, 30, 28, 29) est associé au dispositif d'actionnement (3) et permet de mesurer et de visualiser au moins un déplacement de l'instrument médical (2) en sens longitudinal de l'appareil et/ou sa rotation en sens transversal.

5. Appareil médical selon l'une des revendications précédentes comprenant les caractéristiques suivantes :
- l'appareil médical (1) présente une extrémité proximale qui en permet ou, tout au moins, en facilite le maniement et une extrémité distale qui contient l'instrument médical (2) ;
- l'instrument médical (2) présente une extrémité opérationnelle qui émerge de l'extrémité distale de l'appareil (1) ou que l'on peut en faire sortir, et est, avec sa partie adjacente à cette extrémité opérationnelle et orientée vers l'extrémité proximale de l'appareil (1), déplaçable par un porte-instrument (10) aussi bien en sens longitudinal de l'appareil qu'en rotation transversale par rapport à celui-ci ;
- dans le porte-instrument (10), le (les) premier(s) élément(s) magnétique(s) (18, 19) est (sont) couplé(s) à l'instrument médical (2) ;
- et le (les) premier(s) élément(s) magnétique(s) (18, 19) est (sont) entouré(s) par le (les) second(s) élément(s) magnétique(s) (20, 21), le(s)quel(s) est (sont) déplaçable(s) par rapport au (aux) premier(s) élément(s) magnétique(s) (18, 19) en sens longitudinal de l'appareil et en rotation transversale et agit (agissent) conjointement avec le (les) premier(s) élément(s) magnétique(s) (18, 19).

6. Appareil médical selon la revendication 1, **caractérisé en ce que** le curseur rotatif (7) est déplaçable et rotatif entre le porte-instrument (10) et une prolongation (22) prévue du côté proximal en sens longitudinal de l'appareil et utilisable comme appuie-main, laquelle peut être logée dans le porte-instrument (10).

7. Appareil médical selon l'une des revendications précédentes, **caractérisé en ce que** le (les) premier(s) élément(s) magnétique(s) (18, 19) et le (les) second(s) élément(s) magnétique(s) (20, 21) sont agencés de façon à agir conjointement avec leurs pôles contraires (pôles nord N et pôles sud S).

8. Appareil médical selon l'une des revendications précédentes, **caractérisé en ce que** les éléments magnétiques (18, 19, 20, 21) sont respectivement formés par des barreaux aimantés dont les pôles contraires (pôles nord N et pôles sud S) sont respectivement orientés selon le sens longitudinal de l'appareil.

9. Appareil médical selon l'une des revendications précédentes, **caractérisé en ce que** les éléments magnétiques (18, 19, 20, 21) sont respectivement formés par des aimants ronds dont les pôles contraires (pôles nord N et pôles sud S) sont respectivement orientés transversalement par rapport au sens longitudinal de l'appareil.

10. Appareil médical selon l'une des revendications précédentes, **caractérisé en ce que** les élément magnétique (18, 19, 20, 21) respectifs sont des aimants permanents.

11. Appareil médical selon l'une des revendications précédentes, **caractérisé en ce que** l'instrument médical (2) est un instrument médical à usage endoscopique, un panier pour extraction de calculs ou une anse médicale, par exemple une anse à polypectomie.

12. Appareil médical selon l'une des revendications précédentes, **caractérisé en ce que** le dispositif d'actionnement (3) comprend au moins une cavité (35, 35') dans laquelle le (les) second(s) élément(s) magnétique(s) (20, 21) sont agencés de façon à pouvoir se déplacer longitudinalement.

13. Appareil médical selon la revendication 12, **caractérisé en ce qu'**un élément gradué (33, 33') est déplaçable en direction distale au moyen d'un déplacement longitudinal du (des) second(s) élément(s) magnétique(s) (20, 21) en direction distale.

14. Appareil médical selon l'une des revendications 12 ou 13, **caractérisé en ce qu'**une partie de l'élément gradué (33, 33') associée à un ressort prétendu en direction proximale est agencée dans la cavité (35, 35') en position distale par rapport au(x) second(s) élément(s) magnétique(s) (20, 21).
